# EUROPEAN PATENT APPLICATION

(11) **EP 4 067 881 A1**
(43) Date of publication of application: **05.10.2022**
(21) Application number: 19953820.8
(22) Date of filing: 29.11.2019
(51) Int. Cl.: G01N 21/84, G01N 1/30

(54) **SMEAR PREPARATION DEVICE, AND SAMPLE ANALYSIS SYSTEM AND METHOD**

(71) Applicant: Shenzhen Mindray Bio-Medical Electronics Co., Ltd, Shenzhen, Guangdong 518057 (CN)
(72) Inventor: LI, Xuerong, Shenzhen, Guangdong 518057 (CN); JIANG, Bin, Shenzhen, Guangdong 518057 (CN); YU, Shan, Shenzhen, Guangdong 518057 (CN)
(74) Representative: KIPA AB
(86) International application number: PCT/CN2019/122149
(87) International publication number: WO 2021/102984

(57) **Abstract**

Provided are a smear preparation device, and a sample analysis system and method, the system comprising: a blood analysis device, that tests a sample and obtains a sample analysis result; a smear preparation device, that prepares a smear of the sample; a cell image analysis device, that photographs and analyzes the smear; a conveying device, that comprises a first conveying track and a second conveying track; and a control device, that acquires the sample analysis result of the sample from the blood analysis device, and when the sample analysis result satisfies a preset condition, controlling the smear preparation device to prepare at least two smears of the sample, and controlling the at least two smears to be sent to the cell image analysis device for photographing and analysis. According to the smear preparation device and sample analysis system and method of the present invention, the accuracy and analysis efficiency of cell analysis are improved, and labor costs and resources are greatly reduced by preparing smears of different modes for cell analysis.

## Description

### TECHNICAL FIELD

The present invention relates to the technical field of medical equipment, and in particular to a smear preparation apparatus, sample analysis system and method.

### BACKGROUND

The current blood cell re-examination pipeline includes a blood cell analyzer, a smear staining machine, and a smear reader (a cell morphology analyzer). However, the above modules work independently and cannot systematically solve actual problems of users.

For some special samples, such as samples with low or ultra-low white blood cell (WBC) count values, the number of WBC is extremely small. If the blood smear is prepared and read by the conventional method, even cells of the entire blood smear are scanned in the microscopic region during reading the smear, the set expectations cannot be satisfied. Therefore, an accuracy of classification during the reading and accuracy of statistical results of abnormal cells will be greatly affected.

### SUMMARY

Embodiments of the present invention provide a smear preparation apparatus, a sample analysis system and method, and a computer-readable storage medium, so as to solve at least one of the above problems.

In a first aspect according to the embodiments of the present invention, a sample analysis system is provided. The system includes a blood analysis apparatus, a smear preparation apparatus, a cell image analysis apparatus, a transfer apparatus, and a control apparatus.

The blood analysis apparatus is configured to test a sample and obtain a sample analysis result.

The smear preparation apparatus is configured to prepare a smear for the sample.

The cell image analysis apparatus is configured to photograph and analyze the smear.

The transfer apparatus includes a first transfer track configured to transfer the sample from the blood analysis apparatus to the smear preparation apparatus, and a second transfer track configured to transfer the smear from the smear preparation apparatus to the cell image analysis apparatus.

The control apparatus is communicatively connected with the blood analysis apparatus, the smear preparation apparatus, the cell image analysis apparatus, and the transfer apparatus.

The control apparatus is configured to acquire the sample analysis result of the sample from the blood analysis apparatus; and control the first transfer track to transfer the sample to the smear preparation apparatus, control the smear preparation apparatus to prepare at least two smears of the sample, and control the second transfer track to transfer the at least two smears to the cell image analysis apparatus for photographing and analysis, when the sample analysis result satisfies a preset condition.

In a second aspect according to the embodiments of the present invention, a smear preparation apparatus is provided. The apparatus includes a sampling portion, a smear preparation portion, and a control portion.

The sampling portion is configured to collect a sample.

The smear preparation portion is configured to prepare the sample collected by the sampling portion into a smear. The smear preparation portion includes a slide loading mechanism configured to move a blank slide to an operating position, a sample loading mechanism configured to load the sample onto the blank slide and a smearing mechanism configured to smear the sample on the blank slide. The smear preparation portion is configured with a single-smear mode and a multi-smear mode.

The control portion is electrically connected to the sampling portion and the smear preparation portion.

The control portion is configured to determine an operation mode of the smear preparation portion from the single-smear mode and the multi-smear mode; control the smear preparation portion to prepare a single smear of the sample when it is determined that the operation mode is the single-smear mode; and control the smear preparation portion to prepare a plurality of smears of the sample when it is determined that the operation mode is the multi-smear mode.

In a third aspect according to the embodiments of the present invention, a sample analysis method is provided. The method includes the following operations.

A sample analysis result of a sample is acquired from a blood analysis apparatus.

The sample is transferred to a smear preparation apparatus by a first transfer track; at least two smears of the sample are prepared by the smear preparation apparatus; the at least two smears are transferred to a cell image analysis apparatus by a second transfer track; and cell images of the at least two smears are photographed and analyzed by the cell image analysis apparatus; when the sample analysis result satisfies a preset condition.

In a fourth aspect according to the embodiments of the present invention, there is provided a computer-readable storage medium having stored therein a program that, when executed by a processor, causes the processor to implement the method of the third aspect.

According to the smear preparation apparatus, the sample analysis system and method provided in the embodiments of the present invention, a richer data base is provided for the data source of cell analysis by preparing smears in different modes for cell analysis according to abnormal conditions in the sample analysis result, thereby improving the accuracy and efficiency of the cell analysis, and greatly saving labor costs and resources.

### BRIEF DESCRIPTION OF THE DRAWINGS

In order to explain the embodiments of the present invention or the technical solutions in the related art more clearly, the accompanying drawings used in the description of the embodiments or the related art will be briefly introduced below. It is apparent that the accompanying drawings in the following description are only some embodiments of the present invention. For those of ordinary skill in the art, other drawings can also be obtained according to these drawings without creative efforts.
FIG. 1 illustrates a schematic block diagram of a sample analysis system according to an embodiment of the present invention.
FIGS. 2 and 3 illustrate schematic structure diagrams of a smear preparation apparatus according to embodiments of the present invention, respectively.
FIGS. 4 and 5 illustrate schematic structure diagrams of a cell image analysis apparatus according to embodiments of the present invention, respectively.
FIG. 6 illustrates a schematic structure diagram of a control apparatus according to an embodiment of the present invention.
FIG. 7 illustrates a schematic flowchart of a sample analysis method according to an embodiment of the present invention.

### DETAILED DESCRIPTION

The technical solutions in the embodiments of the present invention will be clearly and completely described below with reference to the accompanying drawings in the embodiments of the present invention. It is apparent that the described embodiments are only a part of the embodiments of the present invention, rather than all the embodiments. Based on the embodiments of the present invention, all other embodiments obtained by those of ordinary skill in the art without creative efforts shall fall within the protection scope of the present invention.

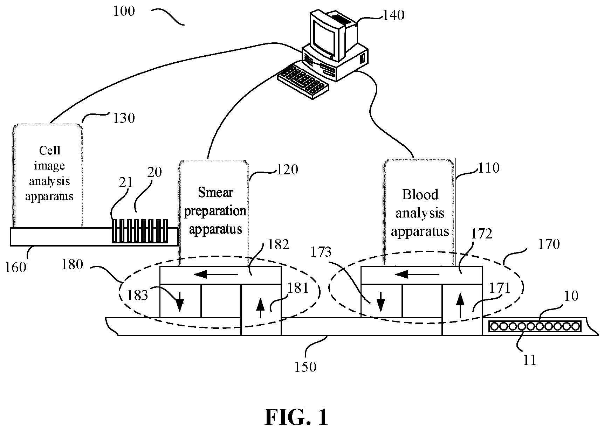

FIG. 1 illustrates a schematic block diagram of a sample analysis system according to an embodiment of the present invention. With reference to FIG. 1, the sample analysis system 100 may include a blood analysis apparatus 110, a smear preparation apparatus 120, a cell image analysis apparatus 130, and a control apparatus 140.

The blood analysis apparatus 110 is configured to detect a sample and obtain a sample analysis result. The smear preparation apparatus 120 is configured to prepare a smear for the sample. The cell image analysis apparatus 130 is configured to photograph and analyze the smear. The control apparatus 140 is connected communicatively with the blood analysis apparatus 110, the smear preparation apparatus 120 and the cell image analysis apparatus 130.

In some embodiments, the blood analysis apparatus 110 may perform blood routine testing on the sample to obtain the blood analysis result, the smear preparation apparatus 120 prepares the smear of the sample, and the cell image analysis apparatus 130 photographs and analyzes images of cells in the smear.

The sample analysis system 100 further includes a transfer apparatus. The transfer apparatus includes a first transfer track 150 configured to transfer the sample from the blood analysis apparatus 110 to the smear preparation apparatus 120, and a second transfer track 160 configured to transfer the smear from the smear preparation apparatus 120 to the cell image analysis apparatus 130.

Specifically, the first transfer track 150 is configured to transfer a test tube rack 10 on which a plurality of test tubes 11 loaded with samples can be placed from the blood analysis apparatus 110 to the smear preparation apparatus 120, and the second transfer track 160 is configured to transfer a slide basket 20 capable of loading a plurality of prepared smears 21 from the smear preparation apparatus 120 to the cell image analysis apparatus 130.

Optionally, the control apparatus 140 communicates with the transfer apparatus in a wired communication manner or a wireless communication manner. The wireless communication manner includes but is not limited to wireless Personal Area Network (PAN, such as Bluetooth), wireless Local Area Network (LAN, such as Wifi), wireless Metropolitan Area Network (MAN, such as WiMAX), or wireless Wide Area Network (WAN, Such as 3G, 4G, and a next-generation mobile communication network).

In some embodiments, the control apparatus 140 may send a first transfer instruction to the transfer apparatus, and after receiving the first transfer instruction, the transfer apparatus controls the first transfer track 150 to transfer the sample from the blood analysis apparatus 110 to the smear preparation apparatus 120. Furthermore, the control apparatus 140 may send the first transfer instruction to the first transfer track 150, and the first transfer track 150 transfers the sample from the blood analysis apparatus 110 to the smear preparation apparatus 120 after receiving the first transfer instruction.

The control apparatus 140 may also send a second transfer instruction to the transfer apparatus, and the transfer apparatus controls the second transfer track 160 to transfer the smear from the smear preparation apparatus 120 to the cell image analysis apparatus 130 after receiving the second transfer instruction. Furthermore, the control apparatus 140 may send the second transfer instruction to the second transfer track 160, and the second transfer track 160 transfers the smear from the smear preparation apparatus 120 to the cell image analysis apparatus 130 after receiving the second transfer instruction.

In some embodiments, the control apparatus 140 may be electrically connected to the transfer apparatus and control the operation of the transfer apparatus. Furthermore, the control apparatus140 may be electrically connected with the first transfer track 150 and the second transfer track 160 and control the operations thereof.

The sample analysis system 100 further includes a feeding mechanism 170 provided for the blood analysis apparatus 110 and a feeding mechanism 180 provided for the smear preparation apparatus 120. As illustrated in FIG. 1, the feeding mechanism 170 includes a loading buffer region 171, a feeding detection region 172, and an unloading buffer region 173, and the feeding mechanism 180 includes a loading buffer region 181, a feeding detection region 182, and an unloading buffer region 183.

When the sample on the test tube rack 10 needs to be transferred to the blood analysis apparatus 110 for detection, the test tube rack 10 is first transferred from the first transfer track 150 to the loading buffer region 171, and then is transferred from the loading buffer region 171 to the feeding detection region 172 for detection by the blood analysis apparatus 110. After the detection, the test tube rack 10 is unloaded from the feeding detection region 172 to the unloading buffer region 173, and finally enters the first transfer track 150 from the unloading buffer region 173.

Similarly, when the samples on the test tube rack 10 need to be microscopically examined, the test tube rack 10 needs to be transferred to the smear preparation apparatus 120 to prepare the smear. The test tube rack 10 is first transferred from the first transfer track 150 to the loading buffer region 181, and then is transferred from the loading buffer region 181 to the feeding detection region 182 to prepare smears by the smear preparation apparatus 120. After the smear preparation is completed, the test tube rack 10 is unloaded from the feeding detection region 182 to the unloading buffer region 183, and finally enters the first transfer track 150 from the unloading buffer region 183. The smear preparation apparatus 120 stores the prepared smear in the slide basket 20, and transfers the slide basket 20 containing the smear to the cell image analysis apparatus 130 through the second transfer track 160. The cell image analysis apparatus 130 photographs and analyzes cells in the sample on the smear.

The sample analysis system 100 further includes a display apparatus (not illustrated in the figure) for displaying a sample test result, which may be provided on the blood analysis apparatus 110, the cell image analysis apparatus 130 or the control apparatus 140, or may be provided separately.

Optionally, the display apparatus may be a touch display screen, a liquid crystal display screen, etc., or may be a display screen on an electronic device such as a mobile phone, a tablet computer, and the like.

In some embodiments, the display apparatus provided in the blood analysis apparatus 110 may display the sample analysis result obtained by the blood analysis apparatus 110 after detecting the sample.

In some embodiments, the display apparatus provided in the cell image analysis apparatus 130 may display a photographed image obtained by the cell image analysis apparatus 130 after photographing cells in the sample on the smear. Furthermore, the cell image analysis apparatus 130 may communicate with the blood analysis apparatus 110 to obtain the sample analysis result from the blood analysis apparatus 110. Furthermore, the cell image analysis apparatus 130 may display the photographed image and the corresponding sample analysis result according to a user's instruction or setting.

In some embodiments, the display apparatus provided in the control apparatus 140 may acquire the sample analysis result from the blood analysis apparatus 110, and/or acquire the photographed image obtained by photographing the cells in the sample on the smear from the cell image analysis apparatus 130, and then display the sample analysis result and/or the photographed image according to the user's instruction or setting.

In some embodiments, a display apparatus provided separately may acquire the sample analysis result from the blood analysis apparatus 110, and/or acquire he photographed image obtained by photographing the cells in the sample on the smear from the cell image analysis apparatus 130, and then display the sample analysis result and/or the photographed image. Alternatively, the display apparatus provided separately may acquire the sample analysis result and/or the photographed image from the control apparatus 140, and then display the sample analysis result and/or the photographed image.

In some embodiments, the blood analysis apparatus 110 may be configured to perform blood routine testing on a sample to be tested, such as a blood sample, to obtain a sample test result. Blood routine parameters or blood test results may include at least one or more of a white blood cell (WBC) five classification result, WBC counting and morphological parameters, a hemoglobin (HGB) test result, or red blood cell (RBC) or blood platelet (PLT) counting and morphological parameters. The blood analysis apparatus 110 generally includes a sampling apparatus, a sample preparation apparatus, an optical detection apparatus, and a controller. The sampling apparatus has a sampling needle configured to collect a blood sample and deliver the collected blood sample to the sample preparation apparatus. The sample preparation apparatus has a reaction cell and a reagent supplying portion configured to store a reagent for reacting with the blood sample and supply the corresponding reagent to the reaction cell as needed. The sample preparation apparatus may include at least one reaction cell, and the at least one reaction cell may be configured such that the blood sample from the sampling portion and the reagent from the reagent supplying portion react to prepare a sample to be tested. The optical detection apparatus includes a light source, a flow cell in which particles of the sample to be tested can flow, and a light detector. The particles in the flow cell are illuminated by light emitted from the light source to generate optical information. The light detector detects the optical information. The controller is configured to control the operations of the sampling apparatus, the sample preparation apparatus and the optical detection apparatus and to acquire the optical information detected by the optical detection apparatus, to further obtain the blood routine testing result of the blood sample. The blood analysis apparatus 110 is connected communicatively with the control apparatus 140 through the controller of the blood analysis apparatus, so as to transmit the sample test result to the control apparatus 140, or may also receive information from the control apparatus 140.

In some embodiments, the controller of the blood analysis apparatus 110 may also be connected communicatively with a controller of the smear preparation apparatus 120 directly, to send the sample analysis result to the smear preparation apparatus 120. The controller of the smear preparation apparatus 120 determines whether it is necessary to smear the sample to be tested.

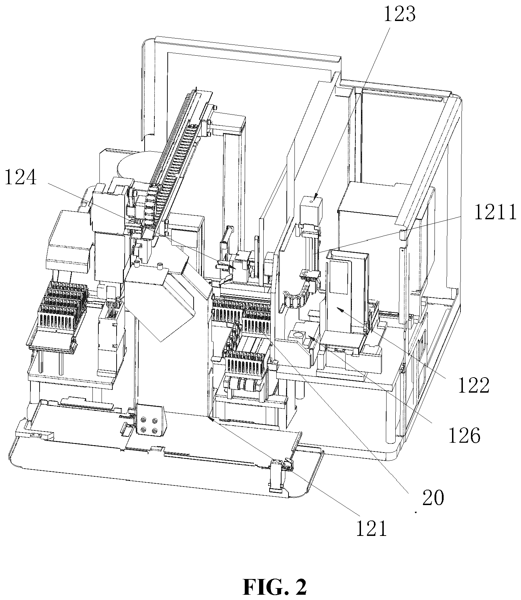

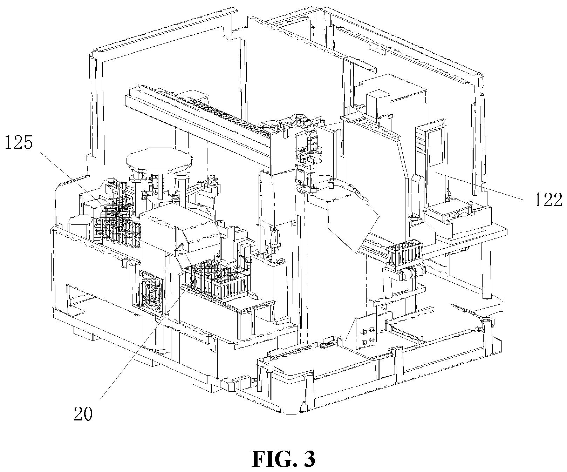

FIG. 2 and FIG. 3 illustrate schematic structure diagrams of a smear preparation apparatus 120 according to embodiments of the present invention, respectively. With reference to FIG. 2 and FIG. 3, the smear preparation apparatus 120 may be configured to prepare a smear of the sample such as blood and body fluid. The smear preparation apparatus includes a sampling mechanism 121 configured to draw the sample, a slide loading mechanism 122 configured to move the slide to a working line, a sample loading mechanism 123 configured to load the sample onto the slide, a smearing mechanism 124 configured to smear the sample on the slide, a drying mechanism (not illustrated in the figure) configured to dry the blood film on the slide, and a staining mechanism 125 configured to stain the slide. The smear preparation apparatus 120 also includes a controller (not illustrated) configured to control operations of the various mechanisms of the smear preparation apparatus 120.

The smear preparation apparatus 120 is connected communicatively with the control apparatus 140 through its controller, to receive a smearing instruction from the control apparatus 140 or send data to the control apparatus 140. In some embodiments, the controller of the smear preparation apparatus 120 may also be connected communicatively with the controller of the blood analysis apparatus 110 directly, to acquire the sample analysis result of the sample to be tested from the blood analysis apparatus 110, so as to determine whether it is necessary to smear the sample to be tested.

After receiving the smearing instruction from the control apparatus 140, the controller of the smear preparation apparatus 120 may send a sampling instruction to the sampling mechanism 121 to control the sampling mechanism 121 to extract the sample. The sampling mechanism 121 first mixes the sample, and then sucks the sample using the sampling apparatus (for example, the sampling needle 1211) in the sampling mechanism 121. Depending on a sample container, the sample suction may be a puncture suction (the sample container has a cover, and the sampling apparatus passes through the cover of the sample container), or may be an open suction (the sample container is open, and the sampling apparatus directly sucks the sample from an opening). When necessary, the controller of the smear preparation apparatus 120 may control the smear preparation device 120 to detect blood sample information, so as to acquire information and compare the information. In some embodiments, the smear preparation apparatus 120 further includes a sample injection mechanism 126 (for example, a micro sample injection mechanism), and the controller of the smear preparation apparatus 120 may send other corresponding instructions to the sample injection mechanism 126 to control the sample injection mechanism 126 to move the test tube put in by the operator towards the sampling apparatus, or the sampling apparatus may also move towards the test tube put in by the operator. In other implementations, the sample injection mechanism 126 may also move the test tube directly towards the sample loading mechanism 123, or the sample loading mechanism 123 may also move towards the test tube put in by the operator. The sample loading mechanism 123 (for example, a blood dripping needle) draws the blood sample directly and then loads the sample. Since there is no need to draw the blood sample through the sampling mechanism 121, the need for the blood sample can be reduced, thereby realizing micro and priority sample injection. Furthermore, when the sampling mechanism 121 completes the sampling, a signal indicating the completion of the sampling may be sent to the controller of the smear preparation apparatus 120.

Correspondingly, the controller of the smear preparation apparatus 120 may control the slide loading mechanism 122 to extract the slide and load the slide to a corresponding position, so as to facilitate the dripping operation. In some embodiments, after the operation of extracting the slide is completed, operations such as left and right detection of the slide and cleaning of the slide may be performed, and then the slide is loaded. The loaded slide can be printed with relevant information, and operations such as front and back surfaces detection can be performed at the same time. It should be noted that the controller of the smear preparation apparatus 120 may control the sampling mechanism 121 to extract the sample and the slide loading mechanism 122 to extract the slide simultaneously, or not simultaneously, which is not limited herein. Furthermore, when the slide loading mechanism 122 finishes extracting the slide and loading the slide to the corresponding position, a signal indicating the completion of slide loading may be sent to the controller of the smear preparation apparatus 120.

After receiving the signal indicating the completion of the sampling and the signal indicating the completion of the slide loading, the controller of the smear preparation apparatus 120 may send a sample loading instruction to the sample loading mechanism 123, and the sample loading mechanism 123 controls the blood dripping needle to drip the sample onto the slide according to the sample loading instruction. Furthermore, when the sample loading mechanism 123 completes the sample loading, a signal indicating the completion of sample loading may be sent to the controller of the smear preparation apparatus 120.

After receiving the signal indicating the completion of sample loading, the controller of the smear preparation apparatus 120 sends a corresponding instruction to the smearing mechanism 124 to perform the smearing operation, and the smearing mechanism 124 smears the blood on the slide into a blood film according to the corresponding instruction. In general, after the smearing operation is completed, the controller of the smear preparation apparatus 120 can control the smear preparation apparatus 120 to dry the blood film on the slide so as to stabilize its shape. In some embodiments, the slide can be driven to turn over before drying the blood film to meet the corresponding requirements. In some embodiments, the dried blood smear may also be tested for determining a drying effect of the blood film. In some embodiments, the dried blood smear may also be tested for determining whether the blood film is spread and whether the spread state meets the requirements. After the smearing is completed, the controller of the smear preparation apparatus 120 may control the smear preparation apparatus 120 to stain the slide (blood smear) (which can be implemented by the staining mechanism 125) or directly output the slide (for example, placing it into the slide basket 20 for output).

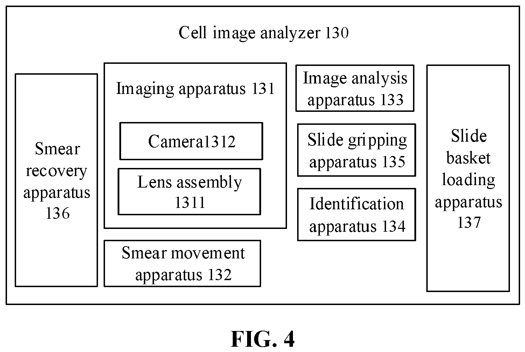

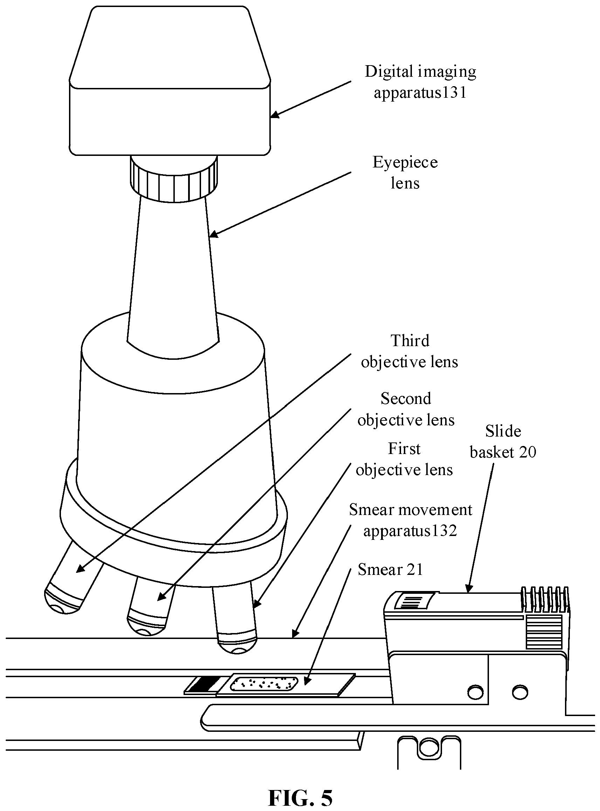

FIGS. 4 and 5 illustrate schematic structure diagrams of a cell image analysis apparatus 130 according to embodiments of the present invention, respectively. With reference to FIGS. 4 and 5, the cell image analysis apparatus 130 includes at least an imaging apparatus 131, a smear movement apparatus 132 and an image analysis apparatus 133. The imaging apparatus 131 includes a camera 1312 and a lens assembly 1311 configured to photograph a cell in a sample smeared on a smear. The smear movement apparatus 132 is configured to move the smear relative to the imaging apparatus 131, so that the imaging apparatus 131 can photograph a cell image of a specific region in the smear. The image analysis apparatus 133 is configured to analyze the cell image of the smear.

As illustrated in FIG. 5, the lens assembly 1311 may include a first objective lens, a second objective lens, and an eyepiece lens. The first objective lens may be, for example, a 10× objective lens, and the second objective lens may be, for example, a 100x objective lens. The lens assembly 1311 may further include a third objective lens, and the third objective lens may be, for example, a 40x objective lens.

The cell image analysis apparatus 130 further includes an identification apparatus 134, a slide gripping apparatus 135 and a smear recovery apparatus 136. The identification apparatus 134 is configured to identify identity information of the smear, the slide gripping apparatus 135 is configured to grip the smear from the identification apparatus 134 to the smear movement apparatus 132 for detection, and the smear recovery apparatus 136 is configured to recover the detected smear.

The cell image analysis apparatus 130 further includes a slide basket loading apparatus 137 configured to load a slide basket containing the smear, and the slide gripping apparatus 135 is further configured to grip the slide in the slide basket loaded on the slide basket loading apparatus 137 to the identification apparatus 134 for identity information identification. The slide basket loading apparatus 137 is connected to the first transfer track 160 so that the smear prepared by the smear preparation apparatus 120 can be transferred to the cell image analysis apparatus 130.

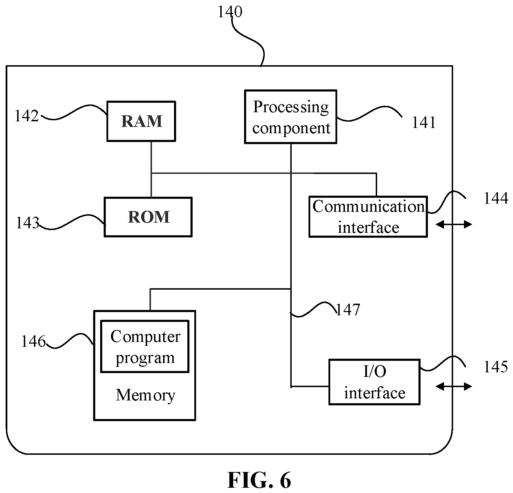

FIG. 6 illustrates a schematic structure diagram of a control apparatus 140 according to an embodiment of the present invention. With reference to FIG. 6, the control apparatus 140 at least includes: a processing component 141, a random access memory (RAM) 142, a read only memory (ROM) 143, a communication interface 144, a memory 146, and an input/output (I/O) interface 145. The processing component 141, the RAM 142, the ROM 143, the communication interface 144, the memory 146, and the I/O interface 145 may communicate with each other via a bus 147.

The processing component 141 may be a central processing unit (CPU), a graphics processing unit (GPU) or other chips with computing capabilities. In practical applications, the processing component 141 may be implemented by software, hardware, firmware or a combination thereof, and may use at least one of a circuit, a single or a plurality of application specific integrated circuits (ASICs), a digital signal processor (DSP), a digital signal processing device (DSPD), a programmable logic device (PLD), a field programmable gate array (FPGA), a CPU, a controller, a microcontroller, or a microprocessor, so that the processing component 141 can be configured to execute some or all of the steps or any combination of the steps in the sample analysis method in the various embodiments of the present invention.

The memory 146 stores various computer programs, such as an operation system and an application program, which are executable by the processor component 141, and data necessary for the execution of the computer programs. In addition, during the sample detection, if data needs to be stored locally, it can be stored in the memory 146.

In some embodiments, the memory 146 may be volatile memory, such as a RAM; or non-volatile memory, such as a ROM, a flash memory, a hard disk drive (HDD), or a solid-state drive (SSD); or a combination of the above types of memory, and the memory 146 is configured to provide instructions and data to the processing component.

The I/O interface 145 is composed of a serial interface such as USB, IEEE1394, or RS-232C, a parallel interface such as SCSI, IDE, or IEEE1284, and an analog signal interface including a digital to analog (D/A) converter, an analog to digital (A/D) converter, and the like. Input devices composed of a keyboard, a mouse, a touch screen or other control buttons are connected to the I/O interface 145, and the user can directly input data to the control apparatus 140 by using the input devices. In addition, the I/O interface 145 can also be connected with a display with a display function, such as a liquid crystal screen, a touch screen, an LED display screen, etc., and the control apparatus 140 can output the processed data, such as analysis data, instrument operating parameters, etc., to the display as image display data for display.

The communication interface 144 may be an interface supporting any communication protocol. The communication interface 144 communicates with an outside world through a network. The control apparatus 140 can transmit data with any apparatus connected to the network with a certain communication protocol through the communication interface 144. It should be understood that the sample analysis system according to the embodiments of the present invention is not limited by the communication interface, both an interface supporting a currently known communication protocol and an interface supporting a communication protocol to be developed in the future can be applied to the sample analysis system in the embodiments of the present invention to implement the operation of communicating with the outside world through the network, which is not limited herein.

When the sample analysis system 100 according to the embodiments of the present invention is operating, a corresponding operation interface may be provided for the operator to operate. The above operation interface may include various controls, such as a selection box or a menu bar, etc., so that the operator can input operation instructions on the operation interface according to an actual use situation, so as to analyze the sample by the sample analysis system 100.

In practical applications, the sample for re-examination may have abnormal cells, and the abnormal may generally include abnormal number and abnormal infection. The number or proportion of some types of cells may be lower than a normal range, for example, in a sample with low or ultra-low WBC counts (the WBC count value ≤ 0.5^{∗}10⁹/L), the number of WBCs in the sample is very small. If only one smear is prepared, the number of such cells in the smear may be even lower. Even all such cells in the entire smear are photographed, the needs of cell analysis, for example, 100 WBCs are required, cannot be met, and an accurate cell morphology analysis result cannot be obtained. Some types of cells do not appear in a normal sample, and they appear only when an infection occurs, such as Plasmodium infection. When performing cell morphological analysis on a sample that may be infected with Plasmodium, a plurality of thick smears and thin smears are manually prepared for comparative analysis, and if only one smear is prepared, it is obviously not sufficient for the cell analysis of the sample that may be infected with Plasmodium. Therefore, when the cells are abnormal, preparation parameters such as the number and thickness of smears that need to be prepared are different.

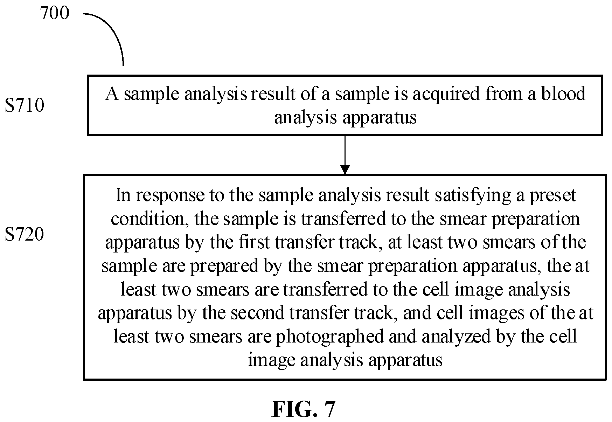

Based on the above considerations, a sample analysis method is provided according to an embodiment of the present invention. Referring to FIG. 7, FIG. 7 illustrates a schematic flowchart of a sample analysis method according to an embodiment of the present invention. As illustrated in FIG. 7, the method 700 includes the following operations.

In S710, a sample analysis result of a sample is acquired from a blood analysis apparatus.

In S720, the sample is transferred to a smear preparation apparatus through a first transfer track when the sample analysis result satisfies a preset condition; at least two smears of the sample are prepared by the smear preparation apparatus; the at least two smears are transferred to a cell image analysis apparatus by a second transfer track; and the at least two smears are photographed and analyzed by the cell image analysis apparatus.

According to the embodiment of the present invention, as illustrated in FIG. 1, the control apparatus 140 may be provided independently of the blood analysis apparatus 110, the smear preparation apparatus 120 and the cell image analysis apparatus 130.

Correspondingly, in the sample analysis system 100 according to the embodiment of the present invention, the control apparatus 140 may acquire the sample analysis result of the sample from the blood analysis apparatus 110, controls, when the sample analysis result satisfies the preset condition, the first transfer track to transfer the sample to the smear preparation apparatus, controls the smear preparation apparatus to prepare at least two smears of the sample, and controls the second transfer track to transfer the at least two smears to the cell image analysis apparatus for photographing and analysis.

The sample analysis result may show a type and number of cells in the sample. When the sample analysis result shows that a certain type of cells is abnormal, further cell analysis is required to determine morphology of the abnormal cell. Traditional sample analysis method cannot meet the requirements of subsequent cell analysis in terms of accuracy and speed. In contrast, in the sample analysis system according to the embodiments of the present invention, the control apparatus 140 can control the smear preparation apparatus 120 to prepare smears with different modes for cell analysis according to an abnormal situation in the sample analysis result, thereby improving the accuracy and efficiency of cell analysis, and saving labor costs and resources greatly.

According to the embodiments of the present invention, the blood analysis apparatus in S710 may refer to any blood analysis apparatus capable of detecting the sample, and may be the blood analysis apparatus 110 in the sample analysis system 100 of the embodiments of the present invention, and may also be other blood analysis apparatuses provided independent of the sample analysis system 100.

Optionally, the operation that the sample analysis result of the sample is acquired from the blood analysis apparatus may include the following operations. The blood analysis apparatus 110 detects the sample and obtains the sample analysis result, and sends the sample analysis result.

Specifically, after the blood analysis apparatus 110 in the sample analysis system 100 detects the sample and obtains the sample analysis result, the blood analysis apparatus 110 sends the sample analysis result to the control apparatus 140, and the control apparatus 140 receives the sample analysis result sent by the blood analysis apparatus 110. When the sample analysis result satisfies the preset condition, the control apparatus 140 controls the first transfer track 150 to transfer the sample to the smear preparation apparatus 120, controls the smear preparation apparatus 120 to prepare at least two smears of the sample, and controls the second transfer track 160 to transfer the at least two smears to the cell image analysis apparatus 130 for photographing and analysis.

According to an embodiment of the present invention, the preset condition in S720 may include an abnormal number of predetermined cells or an abnormal infection.

Optionally, the preset condition includes an abnormal number of predetermined cells in the sample. Furthermore, the abnormal number of predetermined cells means that the number of predetermined cells is lower than a threshold.

Optionally, the predetermined cells are white blood cells.

In some embodiments, the control apparatus 140 may control the smear preparation apparatus to prepare a first number of smears of the sample when the sample analysis result indicates that the number of white blood cells in the sample is less than a first threshold; and the control apparatus 140 may control the smear preparation apparatus to prepare a second number of smears of the sample when the sample analysis result indicates that the number of white blood cells in the sample is greater than or equal to the first threshold and less than a second threshold, and the first number is greater than the second number.

In some embodiments, the control apparatus 140 may control the smear preparation apparatus to prepare a third number of smears of the sample when the sample analysis result indicates that the number of white blood cells in the sample is greater than or equal to the second threshold and less than a third threshold, and the second number is greater than the third number.

In some embodiments, the first number of smears, the second number of smears, or the third number of smears may include a check smear, and the cell analysis result of the check smear is used to test an accuracy of the cell analysis results of other smears.

It should be noted that the first threshold, the second threshold and the third threshold may be different thresholds that can be set as required and are only examples, which do not mean that there are only three thresholds, and the number and values of the thresholds can be set as required, which is not limited herein. Correspondingly, the first number, the second number, and the third number may be different numbers that can be set as required and are only examples, which do not mean that there are only three numbers, and each number may be set as needed, which is not limited herein.

In an embodiment, the blood analysis apparatus 110 detects the sample to obtain the sample analysis result, and sends the sample analysis result to the control apparatus 140. The control apparatus 140 receives the sample analysis result, and compares the number of WBCs in the sample analysis result with the first threshold 0.1^{∗}10⁹/L, the second threshold 0.5^{∗}10⁹/L, and the third threshold 1^{∗}10⁹/L. If the number of WBCs is less than the first threshold 0.1^{∗}10⁹/L, it is determined to prepare the first number of smears, that is, 5 smears of samples; if the number of WBCs is greater than or equal to the first threshold 0.1^{∗}10⁹/L and less than the second threshold 0.5^{∗}10⁹/L, then it is determined to prepare the second number of smears, that is, 3 smears of samples; if the number of WBCs is greater than or equal to the second threshold 0.5^{∗}10⁹/L and less than the third threshold 1^{∗}10⁹/L, it is determined to prepare a third number of smears, that is, 2 smears of samples. Furthermore, if the number of WBCs is greater than or equal to the third threshold 1^{∗}10⁹/L, it means that the sample analysis result indicates that there is no abnormal number of white blood cells in the sample, and only one smear needs to be prepared to meet the requirements of cell morphology analysis. The control apparatus 140 determines whether the number of white blood cells is abnormal and the number of smears to be prepared according to the sample analysis result, sends a smearing instruction including the number of smears to the smear preparation apparatus 120, and controls the first transfer track to transfer the sample from the blood analysis apparatus 110 to the smear preparation apparatus 120. The smear preparation apparatus 120 prepares the number of smears of the sample according to the smearing instruction, and after the smear preparation apparatus 120 completes the preparation of the number of smears, a preparation completion instruction may be sent to the control apparatus 140. Then, the control apparatus 140 controls the second transfer track to transfer all the prepared smears to the cell image analysis apparatus 130 for photographing and analysis, and sends a corresponding photographing condition to the cell image analysis apparatus 130. Next, the cell image analysis apparatus 130 photographs and analyzes the smears of the sample according to the corresponding photographing condition.

Optionally, the preset condition includes abnormal infection of the sample. Furthermore, the abnormal infection of the sample includes Plasmodium infection.

Optionally, the control apparatus 140 may further control the smear preparation apparatus to prepare at least two smears of the sample under different preparation conditions when the sample analysis result satisfies the preset condition.

Optionally, the different preparation conditions include different smearing thicknesses. Furthermore, the control apparatus 140 may further control the smear preparation apparatus 120 to prepare at least two smears of the sample with different smearing thicknesses when the sample analysis result satisfies the preset condition. Furthermore, the control apparatus 140 may further control the smear preparation apparatus 120 to prepare the at least two smears of the sample with different thicknesses under different preparation parameters.

In some embodiments, the different preparation parameters may include at least one of different blood dripping volumes, different swearing angles, and different swearing speeds. The larger the blood dripping volume, the larger the smearing angle, and the faster the smearing speed, the thicker the film on the prepared smear. The smaller the blood dripping volume, the smaller the smearing angle, and the slower the smearing speed, the thinner the film on the prepared smear.

Optionally, the different preparation conditions may further include different staining conditions.

Optionally, the different staining conditions include different pre-staining treatment conditions. Furthermore, the pre-staining treatment condition includes the treatment of maintaining morphology of red blood cells or the treatment of lysing red blood cells. In some embodiments, the treatment of maintaining the morphology of the red blood cells may include a methanol fixation treatment. In some embodiments, the treatment of lysing the red blood cells may include a distilled water hemolysis treatment.

In some embodiments, the control apparatus may further control the smear preparation apparatus to prepare a first smear of the sample under a first staining condition and to prepare a second smear of the sample under a second staining condition, when the sample analysis result indicates that Plasmodium infection occurs in the sample, a sample smearing thickness of the first smear is smaller than the sample smearing thickness of the second smear. The first staining condition includes a methanol fixation treatment on the first smear, and the second staining condition includes a distilled water hemolysis treatment on the second smear.

Specifically, the blood analysis apparatus 110 detects the sample to obtain the sample analysis result, and sends the sample analysis result to the control apparatus 140. The control apparatus 140 receives the sample analysis result, and determines a presence of Plasmodium infection in the sample according to the sample analysis result. The control apparatus 140 determines that at least one thin smear and at least one thick smear need to be prepared for the sample, and sends a smearing instruction including preparing at least one thin smear and at least one thick smear to the smear preparation apparatus 120. The smear preparation apparatus 120 prepares at least one thin smear under the first staining condition, and prepares at least one thick smear under the second staining condition. The smear preparation apparatus 120 may first prepare a thin smear or first prepare a thick smear, the thin smear and thick smear may also be prepared alternately to obtain a plurality of thin smears and thick smears. When the smear preparation apparatus 120 prepares a thin smear, the slide loading mechanism moves a blank slide to an operating position, the sample loading mechanism loads a first blood dripping volume of the sample onto the blank slide, and the smearing mechanism smears the sample on the slide at a first smearing angle and first smearing speed. Then, the sample on the slide is fixed with methanol before staining to maintain the morphology of the red blood cells in the sample on the slide. The morphology of Plasmodium in the red blood cell remains intact, which facilitates determination of the species, morphology and life stage of Plasmodium. Then, after the fixation treatment before staining, the sample on the slide is dried, and then stained with Giemsa stain or Wright stain to obtain the thin smear. When the smear preparation apparatus120 prepares a thick smear, the slide loading mechanism moves a blank slide to the operating position, the sample loading mechanism loads a second blood dripping volume of the sample onto the blank slide, and the second blood dripping volume is larger than the first blood dripping volume. The smearing mechanism smears the sample on the slide with a second smearing angle and a second smearing speed, the second smearing angle is greater than the first smearing angle, and the second smearing speed is faster than the first smearing speed. Then, distilled water is used to perform hemolysis treatment on the sample on the slide before staining to lyse the red blood cells, which facilitates counting the number of Plasmodium. Then, after the distilled water hemolysis treatment before staining, the sample on the slide is dried, and stained with Giemsa stain or Wright stain to obtain the thick smear.

In some embodiments, the sample may be in the form of a thin film in a thin smear and in the form of a disk in a thick smear. The cells on the thin smear are laid flat on the slide, which is convenient for observing the species, morphology and life stage of Plasmodium, and the cells on the thick smear are stacked on the slide, which is convenient for comprehensive counting of Plasmodium.

Optionally, the different staining conditions include different staining parameters. Furthermore, the staining parameter may include: a staining time, a staining temperature, types of reagents in the stain and their proportions. The type of reagent may include a staining reagent or a buffering reagent. For example, the i-th staining parameter may include the i-th staining time, the i-th staining temperature, and a ratio of the reagent Sᵢ₁ to the reagent Sᵢ₂ in the i-th stain as Pᵢ₁:Pᵢ₂, i being a positive integer. The longer the staining time, the more the white blood cells bind to the stain, and the darker the white blood cells are. In the stain containing the staining reagent and the buffering reagent, the ratio of the staining reagent to the buffering reagent can be adjusted. The higher the ratio of the staining reagent to the buffering reagent, the darker the white blood cells stained, and vice versa. The higher the staining temperature, the darker the cells stained.

In some embodiments, the smear preparation apparatus 120 may preset a plurality of different staining parameters, and the control apparatus 140 may automatically select the required staining parameters for staining the sample according to the sample analysis result. The staining parameters may be set or updated as needed (such as increase, decrease, change, etc.), and the staining parameters may be set or updated directly in the smear preparation apparatus 120 and saved locally, and then the staining parameters are sent to the control apparatus 140. In an example, the staining parameters may also be set or updated in the control apparatus 140, and then the staining parameters are sent to the smear preparation apparatus 120 and saved locally. The control apparatus 140 may save the staining parameters and keep synchronization with the staining parameters in the smear preparation apparatus 120. Optionally, the control apparatus 140 may not save the staining parameters, and obtain them from the smear preparation apparatus 120 when necessary.

In some embodiments, the staining parameter may further include a staining mode. The staining mode includes drip staining, dip staining of a single smear, and jar staining of multiple smears. When the smear preparation apparatus 120 is in the drip staining mode, the staining mechanism 125 can drip the stain onto a single smear for staining. When a plurality of smears are prepared, the staining time and stains required for respective smears are different. Therefore, when the staining mechanism 125 stains a plurality of smears with the drip staining mode, one or more of the stain, staining time, and staining temperature may be changed at any time according to the needs of each smear. When the smear preparation apparatus 120 is in the mode for dip staining of a single smear, the staining mechanism 125 may put the smear into a staining box, and add the corresponding stain to the staining box for staining. Similarly, when the staining mechanism 125 stains a plurality of smears with the mode for dip staining of a single smear, one or more of the stain, staining time and staining temperature may be changed at any time according to the needs of each smear. When the smear preparation device 120 is in the mode for jar staining of multiple smears, the staining mechanism 125 can stain a plurality of smears in one jar. At this time, only the staining time can be adjusted until the staining of the plurality of smears is completed. After that, the stain and staining time in the jar can be changed to stain a plurality of smears in the next staining process. Furthermore, the smear preparation apparatus 120 can be switched between different staining modes.

In an embodiment, different staining parameters (the number of the staining parameters is a) are preset in the smear preparation apparatus 120, where b0 is a normal staining parameter, and b0 may include a first staining time, a first staining temperature, a first stain and a drip staining mode. The smear preparation apparatus 120 may send the preset different staining parameters to the control apparatus 140. After receiving the sample analysis result, the control apparatus 140 determines that the number of white blood cells in the sample analysis result is low, the sample belongs to a low-value white blood cell sample, and m smears need to be prepared. One smear is stained normally, that is, stained by using the normal staining parameter b0, and the other smears are stained with the staining parameter b1, which may include a second staining time, a second staining temperature, a second stain and a drip staining mode. The control apparatus 140 sends the smearing instruction to the smear preparation apparatus 120, and the smearing instruction includes: preparing m smears of the sample, the staining parameter for one smear is b0, and the staining parameter for the other m-1 smears is b1. The smear preparation apparatus 120 receives the smearing instruction, loads m slides and adds the sample to the m slides, performs staining according to the staining parameters in the smearing instruction, and then washes and air-dries the stained smears to obtain m smears of the sample.

In some embodiments, the control apparatus 140 may further determine the number of smears to be prepared and the staining parameter for the prepared smear, including the staining parameter and pre-staining treatment condition, according to the sample analysis result.

In an embodiment, c different staining parameters are preset in the smear preparation apparatus 120, where the first staining parameter d0 may include the treatment of maintaining the morphology of red blood cells, a third staining time, a third staining temperature, a third stain and a drip staining mode, and the second staining parameter d1 may include the treatment of lysing red blood cells, a fourth staining time, a fourth staining temperature, a fourth stain and a drip staining mode. The smear preparation apparatus 120 may send the c preset different staining parameters to the control apparatus 140. After receiving the sample analysis result, the control apparatus 140 determines that the sample is infected with Plasmodium according to the sample analysis result, m1 thick smears and m2 thin smears are required to be prepared, m1 thick smears are stained with the second staining parameter d1, and m2 thin smears are stained with the first staining parameter d0. Then the control apparatus 140 sends a smearing instruction to the smear preparation apparatus 120, and the smearing instruction includes: preparing m1 thick smears and m2 thin smears of the sample, the preparation conditions of m1 thick smears including the first smearing thickness and the second staining parameter d1, and the preparation conditions of m2 thin smears including the second smear thickness and the first staining parameter d0. The smear preparation apparatus 120 receives the smearing instruction, loads m1 slides and adds the sample on the m1 slides. After that, smearing is performed based on the first smearing thickness, then the smears are stained with the second staining parameter d1, and then the stained smears are washed and air-dried to obtain m1 thick smears of the sample. The smear preparation apparatus 120 further loads m2 slides and adds the sample on the m2 slides. Then, smearing is performed based on the second smearing thickness, the smears are stained with the first staining parameter d0, and then the stained smears are washed and air-dried to obtain m2 thin smears of the sample.

Optionally, the control apparatus 140 may further be configured to control the cell image analysis apparatus to photograph the at least two smears under different photographing conditions when the sample analysis result satisfies the preset condition.

Optionally, the different photographing conditions include at least one of different photographing positions, different photographing areas, different photographing numbers, or different photographing lenses.

In some embodiments, the photographing position includes at least one of: a tail, a head, a body, a head-body junction, a body-tail junction, an edge, or a designated part of the sample on the smear.

In some embodiments, the photographing area includes an area of the sample on the smear photographed each time. In an embodiment, when photographing is performed with an oil immersion lens, the area of the sample seen through the oil immersion lens may be referred to as a field of view of the oil immersion lens. When the oil immersion lenses with different magnifications are used to observe the sample, the fields of view of the oil immersion lenses will be different. The larger the magnification of the oil immersion lens, the smaller the field of view of the oil immersion lens.

In an embodiment, images of at least 200 fields of view of the oil immersion lens are required to be photographed for the thick smear.

In some embodiments, the different photographing lenses include different optical parameters of the photographing lenses. Furthermore, the optical parameter includes magnification. As illustrated in FIG. 5, the different photographing lenses may include a first objective lens, a second objective lens and an eyepiece lens. The first objective lens may be, for example, a 10× objective lens, and the second objective lens may be, for example, a 100x objective lens. The photographing lenses may further include a third objective lens, and the third objective lens may be, for example, a 40x objective lens.

In some embodiments, the photographed number includes a number of cells photographed in the smear. In some embodiments, the cell image analysis apparatus 130 may have a default photographing condition. When the cell image analysis apparatus 130 is instructed to perform photographing without indicating the photographing condition in the photographing instruction received by the cell image analysis apparatus 130, the cell image analysis apparatus 130 may photograph and analyze the smear sent to the cell image analysis apparatus 130 by the second transfer track 160 based on the default photographing condition.

In an embodiment, when the preset condition includes that the number of white blood cells is lower than a threshold, the blood analysis apparatus 110 detects the sample to obtain the sample analysis result, and sends the sample analysis result to the control apparatus 140. The control apparatus 140 receives the sample analysis result, determines, according to the sample analysis result, that the number of white blood cells is lower than the threshold, determines the number of smears to be prepared (such as three) according to different threshold ranges, sends a smearing instruction including preparing three smears to the smear preparation apparatus 120, and controls the first transfer track to transfer the sample from the blood analysis apparatus 110 to the smear preparation apparatus 120. The smear preparation apparatus 120 prepares three smears of the sample according to the smearing instruction. Then, the control apparatus 140 controls the second transfer track to send the prepared three smears to the cell image analysis apparatus 130 for photographing and analysis, and sends a photographing instruction to the cell image analysis apparatus 130 that does not include a photographing condition, the photographing instruction instructing the cell image analysis apparatus 130 to start photographing and analyzing the smear of the sample. Next, the cell image analysis apparatus 130 photographs the smear of the sample based on the default photographing condition to obtain a cell image, and the cell image analysis apparatus 130 performs identification and classification based on the cell image to obtain a corresponding identification and classification result as the cell analysis result. The cell image analysis apparatus 130 saves the cell analysis result locally and/or sends it to the control apparatus 140 for storage.

In some embodiments, the cell image analysis apparatus 130 may also photograph and analyze the smear sent to the cell image analysis apparatus 130 by the second transfer track 160 based on the default photographing condition when no photographing instruction is received.

It should be noted that the default photographing condition may be set in the cell image analysis apparatus 130 when it leaves the factory, or may be set or changed by an operator as required, which is not limited herein.

In some embodiments, the control apparatus 140 may further be configured to: control the smear preparation apparatus to prepare the first smear and the second smear of the sample when the sample analysis result indicates that Plasmodium infection occurs in the sample, the sample smearing thickness of the first smear being smaller than the sample smearing thickness of the second smear; and control the cell image analysis apparatus to photograph a cell image of a first area of the first smear and photograph a cell image of a second area of the second smear, the first area being smaller than the second area.

In an embodiment, when the preset condition includes Plasmodium infection, the blood analysis apparatus 110 detects the sample to obtain the sample analysis result, and sends the sample analysis result to the control apparatus 140. The control apparatus 140 receives the sample analysis result, determines that Plasmodium infection occurs in the sample according to the sample analysis result, sends a smearing instruction including preparing a thick smear and a thin smear to the smear preparation apparatus 120, and controls the first transfer track to transfer the sample from the blood analysis apparatus 110 to the smear preparation apparatus 120. The smear preparation apparatus 120 prepares the thick smear and the thin smear of the sample according to the smearing instruction. Then, the control apparatus 140 controls the second transfer track to send the prepared thick smear and thin smear to the cell image analysis apparatus 130 for photographing and analysis, and sends a photographing instruction including the photographing condition to the cell image analysis apparatus 130, the photographing condition including that: the photographing position of the thick smear includes at least the edge of the sample on the smear, an objective lens with a lower magnification, such as the first objective lens with a magnification of 10 times, is adopted, and images of at least m fields of view of the objective lens are photographed to cover the entire sample region on the thick smear, m being a positive integer; the photographing position of the thin smear includes at least the tail, head, body, head-body junction, body-tail junction, edge of the sample on the smear, an objective lens with a higher magnification, such as the second objective lens with a magnification of 100 times, is adopted, and images of at least n fields of view of the objective lens are photographed to cover the entire sample region on the thin smear, n being a positive integer. Next, the cell image analysis apparatus 130 photographs and analyzes the thick smear and the thin smear based on the photographing instruction including the photographing condition. Specifically, when the cell image analysis apparatus 130 photographs the thick smear, the cell image analysis apparatus 130 switches the objective lens to the first objective lens, and photographs, from one end of the sample on the thick smear, thick smear cell images of at least m fields of view of the objective lens that can cover the entire sample region on the thick smear based on a preset photographing sequence. When photographing the thin smear, the cell image analysis apparatus 130 switches the objective lens to the second objective lens, and photographs, from the head of the sample on the thin smear with a photographing angle of view from top to bottom and in the order of the field of view from left to bottom, the head, head-body junction, body, body-tail junction, tail, and edge of the sample, to obtain thin smear cell images of at least n fields of view of the objective lens covering the entire sample region on the thin smear. Next, the user can determine the species and stage of Plasmodium and estimate a concentration of Plasmodium in the sample by observing and analyzing the thin smear images. The user counts the number of Plasmodium when a certain number of white blood cells are observed based on the smear. The cell image analysis apparatus 130 can calculate the number of Plasmodium per cubic millimeter of the sample based on the total number of white blood cells and the counted number of Plasmodium or the estimated concentration of Plasmodium in the sample analysis result. The cell image analysis apparatus 130 may output a cell analysis result, including the number of Plasmodium per cubic millimeter of the sample, the concentration of Plasmodium in the sample, and the like. The cell image analysis apparatus 130 may store the cell analysis result locally and/or send it to the control apparatus 140 for storage.

It should be understood that the preset photographing sequence may be set as required, which is not limited herein. It should be noted that the above embodiments are only examples, and the photographing conditions of the thick smear and the thin smear are not limited to the above conditions. For example, the thick smear and the thin smear can be photographed by using the objective lenses with the same magnification, or can be photographed by using the objective lenses with different magnifications, which is not limited herein. Optionally, the control apparatus 140 may further control the smear preparation apparatus 120 to prepare a smear of the sample and control the second transfer track 160 to send the one smear to the cell image analysis apparatus 130 for photographing and analysis, when the sample analysis result does not satisfy the preset condition.

Specifically, when the sample analysis result does not satisfy the preset conditions, it means that there is no abnormal cell in the sample, and only one smear needs to be prepared to meet the needs of cell morphology identification. Then, the control apparatus 140 sends a smearing instruction including preparing one smear to the smear preparation apparatus 120, and the smear preparation apparatus 120 can prepare one smear based on the default preparation condition. The default preparation condition may be set when the smear preparation apparatus leaves the factory, or may be set or changed by the operator as required, which is not limited herein.

According to an embodiment of the present invention, when the control apparatus 140 is set independently of the blood analysis apparatus 110, the smear preparation apparatus 120 and the cell image analysis apparatus 130, the control apparatus 140 may communicate with at least one of the blood analysis apparatus 110, the smear preparation apparatus 120 and the cell image analysis apparatus 130.

In some embodiments, the blood analysis apparatus 110 includes a first controller, and the control apparatus 140 may communicate with the first controller of the blood analysis apparatus 110. The first controller sends the sample analysis results of the blood analysis apparatus 110 to the control apparatus 140. The control apparatus 140 receives the sample analysis result sent by the first controller, and determines the number of smears to be prepared for the sample, the corresponding preparation condition, and the corresponding photographing condition according to the sample analysis result. The control apparatus 140 sends the smearing instruction including the number of smears to be prepared and the corresponding preparation condition, and the photographing instruction including the photographing condition to the first controller, and the first controller sends the smearing instruction to the smear preparation apparatus 120, and sends the photographing instruction to the cell image analysis apparatus 130. The control apparatus 140 or the first controller may control the first transfer track 150 to send the sample to the smear preparation apparatus 120, and the smear preparation apparatus 120 prepares the smear of the sample according to the smearing instruction after receiving the smearing instruction. The control apparatus 140 or the first controller may control the second transfer track 160 to send the smear to the cell image analysis apparatus 130. After receiving the photographing instruction, the cell image analysis apparatus 130 photographs and analyzes the smear transferred by the second transfer track 160 according to the photographing instruction. Furthermore, the cell image analysis apparatus 130 sends the analysis result of the smear to the first controller, or sends the analysis result of the smear to the smear preparation apparatus 120 and the smear preparation apparatus 120 sends the analysis result to the first controller. Then the first controller sends the analysis result to the control apparatus 140.

In some embodiments, the smear preparation apparatus 120 includes a second controller, and the control apparatus 140 can communicate with the second controller of the smear preparation apparatus 120. The second controller obtains the sample analysis result from the first controller of the blood analysis apparatus 110, and sends the sample analysis result to the control apparatus 140. The control apparatus 140 determines the number of smears to be prepared for the sample, the corresponding preparation condition, and the corresponding photographing condition according to the sample analysis results. The control apparatus 140 sends the smearing instruction including the number of smears to be prepared and the preparation condition, and the photographing instructions including the photographing condition to the second controller, and the second controller sends the photographing instruction to the cell image analysis apparatus 130. The control apparatus 140 or the second controller may control the first transfer track 150 to transfer the sample to the smear preparation apparatus 120, and the second controller controls the smear preparation apparatus 120 to prepare the smear of the sample according to the smearing instruction after receiving the smearing instruction. The control apparatus 140 or the second controller may control the second transfer track 160 to transfer the smear to the cell image analysis apparatus 130. After receiving the photographing instruction, the cell image analysis apparatus 130 photographs and analyzes the smear transferred by the second transfer track 160 according to the photographing instruction. Furthermore, the cell image analysis apparatus 130 sends the analysis result of the smear to the smear preparation apparatus 120, and the smear preparation apparatus 120 sends the analysis result to the control apparatus 140.

In some embodiments, the control apparatus 140 may communicate with the first controller of the blood analysis apparatus 110 and the second controller of the smear preparation apparatus 120. The control apparatus 140 acquires the sample analysis result from the first controller, and determines the number of smears to be prepared for the sample and the corresponding preparation condition, as well as the corresponding photographing condition according to the sample analysis result. The control apparatus 140 sends the smearing instruction including the number of smears to be prepared and the preparation condition, and the photographing instruction including the photographing condition to the second controller, and the second controller sends the photographing instruction to the cell image analysis apparatus 130. The control apparatus 140 or the second controller may control the first transfer track 150 to transfer the sample to the smear preparation apparatus 120, and the second controller controls the smear preparation apparatus 120 to prepare the smear of the sample according to the smearing instruction after receiving the smearing instruction. The control apparatus 140 or the second controller may control the second transfer track 160 to transfer the smear to the cell image analysis apparatus 130. The cell image analysis apparatus 130 photographs and analyzes the smear transferred by the second transfer track 160 according to the photographing instruction after receiving the photographing instruction. Furthermore, the cell image analysis apparatus 130 sends the analysis result of the smear to the first controller and/or the second controller, and the first controller and/or the second controller sends the analysis result of the smear to the control apparatus 140.

In some embodiments, the cell image analysis apparatus 130 includes a third controller, and the control apparatus 140 may communicate with the third controller of the cell image analysis apparatus 130. The blood analysis apparatus 110 sends the sample analysis result to the third controller, and the third controller sends the sample analysis result to the control apparatus 140. The control apparatus 140 determines the number of smears to be prepared for the sample, the corresponding preparation condition, and the corresponding photographing condition according to the sample analysis result. The control apparatus 140 sends the smearing instruction including the number of smears to be prepared and the preparation condition, and the photographing instruction including the photographing condition to the third controller, and the third controller sends the smearing instruction to the smear preparation apparatus 120. The control apparatus 140 or the third controller may control the first transfer track 150 to transfer the sample to the smear preparation apparatus 120, and the smear preparation apparatus 120 prepares the smear of the sample according to the smearing instruction after receiving the smearing instruction. The control apparatus 140 or the cell image analysis apparatus 130 may control the second transfer track 160 to transfer the smear to the cell image analysis apparatus 130. After receiving the photographing instruction, the cell image analysis apparatus 130 photographs and analyzes the smear transferred by the second transfer track 160 according to the photographing instruction. Furthermore, the third controller sends the analysis result of the smear to the control apparatus 140.

In some embodiments, the control apparatus 140 may communicate with the first controller of the blood analysis apparatus 110 and the third controller of the cell image analysis apparatus 130. The control apparatus 140 acquires the sample analysis result from the first controller of the blood analysis apparatus 110, and determines the number of smears to be prepared for the sample and the corresponding preparation condition, as well as the corresponding photographing condition according to the sample analysis result. The control apparatus 140 sends the smearing instruction including the number of smears to be prepared and the preparation condition, and the photographing instruction including the photographing condition to the third controller, and the third controller sends the smearing instruction to the smear preparation apparatus 120. The control apparatus 140 or the third controller may control the first transfer track 150 to transfer the sample to the smear preparation apparatus 120, and the smear preparation apparatus 120 prepares the smear of the sample according to the smearing instruction after receiving the smearing instruction. The control apparatus 140 or the third controller may control the second transfer track 160 to transfer the smear to the cell image analysis apparatus 130, and after receiving the photographing instruction, the third controller controls the cell image analysis apparatus 130 to photograph and analyze the smear transferred by the second transfer track 160 according to the photographing instruction. Furthermore, the third controller sends the analysis result of the smear to the control apparatus 140.

In some embodiments, the control apparatus 140 may communicate with the second controller of the smear preparation apparatus 120 and the third controller of the cell image analysis apparatus 130. The second controller or the third controller acquires the sample analysis result from the blood analysis apparatus 110, and sends the sample analysis result to the control apparatus 140. The control apparatus 140 determines the number of smears to be prepared for the sample and the corresponding preparation condition, as well as the corresponding photographing condition according to the sample analysis result. The control apparatus 140 sends the smearing instruction including the number of smears to be prepared and the preparation condition to the smear preparation apparatus 120, and sends the photographing instruction including the photographing condition to the cell image analysis apparatus 130. The control apparatus 140 or the second controller or the third controller may control the first transfer track 150 to transfer the sample to the smear preparation apparatus 120, and the second controller controls the smear preparation apparatus 120 to prepare the smear of the sample according to the smearing instruction after receiving the smearing instruction. The control apparatus 140 or the second controller or the third controller may control the second transfer track 160 to transfer the smear to the cell image analysis apparatus 130, and after receiving the photographing instruction, the third controller controls the cell image analysis apparatus 130 to photograph and analyze the smear transferred by the second transfer track 160 according to the photographing instruction. Furthermore, the third controller sends the analysis result of the smear to the control apparatus 140.

In some embodiments, the control apparatus 140 may communicate with the first controller in the blood analysis apparatus 110, the second controller in the smear preparation apparatus 120 and the third controller in the cell image analysis apparatus 130. The first controller sends the sample analysis result obtained by testing the sample by the blood analysis apparatus 110 to the control apparatus 140. The control apparatus acquires the sample analysis result, and determines the number of smears to be prepared for the sample and the corresponding preparation condition, as well as the corresponding photographing condition according to the sample analysis result. The control apparatus 140 sends the smearing instruction including the number of smears to be prepared and the preparation condition to the second controller and sends the photographing instruction including the photographing condition to the third controller. The control apparatus 140 may control the first transfer track 150 to transfer the sample to the smear preparation apparatus 120, and the second controller controls the smear preparation apparatus 120 to prepare the smear of the sample according to the smearing instruction after receiving the smearing instruction. The control apparatus 140 may control the second transfer track 160 to transfer the smear to the cell image analysis apparatus 130, and after receiving the photographing instruction, the third controller controls the cell image analysis apparatus 130 to photograph and analyze the smear transferred by the second transfer track 160 according to the photographing instruction. Furthermore, the third controller sends the analysis result of the smear to the control apparatus 140.

According to an embodiment of the present invention, the control apparatus 140 may also be arranged in the blood analysis apparatus 110, the smear preparation apparatus 120 or the cell image analysis apparatus 130.

Optionally, the control apparatus 140 is arranged in the blood analysis apparatus 110. The blood analysis apparatus 110 tests the sample and obtains the sample analysis result, and determines the number of smears to be prepared for the sample and the corresponding preparation condition according to the sample analysis result. The blood analysis apparatus 110 sends a smearing instruction including the number of smears to be prepared and the preparation condition to the smear preparation apparatus 120, and sends a photographing instruction including the photographing condition to the cell image analysis apparatus 130. The blood analysis apparatus 110 may control the first transfer track 150 to transfer the sample to the smear preparation apparatus 120, and the smear preparation apparatus 120 prepares a smear of the sample according to the smearing instruction after receiving the smearing instruction. The blood analysis apparatus 110 may also control the second transfer track 160 to transfer the smear to the cell image analysis apparatus 130. The cell image analysis apparatus 130 photographs and analyzes the smear transferred by the second transfer track 160 according to the photographing instruction after receiving the photographing instruction.

Optionally, the control apparatus 140 is arranged in the smear preparation apparatus 120. The blood analysis apparatus 110 tests the sample and obtains the sample analysis result, and sends the sample analysis result to the smear preparation apparatus 120. The smear preparation apparatus 120 may control the first transfer track 150 to transfer the sample to the smear preparation apparatus 120. After receiving the sample analysis result, the smear preparation apparatus 120 determines the number of smears to be prepared for the sample and the corresponding preparation condition according to the sample analysis result, and prepares the smear of the sample. The smear preparation apparatus 120 may further control the second transfer track 160 to transfer the smear to the cell image analysis apparatus 130, and send a photographing instruction including the photographing condition to the cell image analysis apparatus 130. The cell image analysis apparatus 130 photographs and analyzes the smear transferred by the second transfer track 160 according to the photographing instruction after receiving the photographing instruction.

Optionally, the control apparatus 140 is arranged in the cell image analysis apparatus 130. The blood analysis apparatus 110 tests the sample and obtains the sample analysis result, and sends the sample analysis result to the cell image analysis apparatus 130. After receiving the sample analysis result, the cell image analysis apparatus 130 determines the number of smears to be prepared for the sample, the corresponding preparation condition, and the corresponding photographing condition according to the sample analysis result. The cell image analysis apparatus 130 sends a smearing instruction including the number of smears to be prepared and the preparation condition to the smear preparation apparatus 120. The cell image analysis apparatus 130 may control the first transfer track 150 to transfer the sample to the smear preparation apparatus 120, and after receiving the smearing instruction, the smear preparation apparatus 120 prepares the smear of the sample according to the smearing instruction. The cell image analysis apparatus 130 may further control the second transfer track 160 to transfer the smear to the cell image analysis apparatus 130, and the cell image analysis apparatus 130 photographs and analyzes the smear transferred by the second transfer track 160 according to the photographing instruction.

According to an embodiment of the present invention, a smear preparation apparatus is further provided. The apparatus includes a sampling portion, a smear preparation portion, and a control portion.

The sampling portion is configured to collect a sample.

The smear preparation portion is configured to prepare the sample collected by the sampling portion into a smear. The smear preparation portion includes a slide loading mechanism configured to move a blank slide to an operating position, a sample loading mechanism configured to load the sample onto the blank slide and a smearing mechanism configured to smear the sample on the blank slide. The smear preparation portion includes a single-smear mode and a multi-smear mode.

The control portion is electrically connected to the sampling portion and the smear preparation portion, and is configured as follows.

The control portion is configured to determine an operation mode of the smear preparation portion from the single-smear mode and the multi-smear mode; and control the smear preparation portion to prepare a single smear of the sample when it is determined that the operation mode is the single-smear mode; and control the smear preparation portion to prepare a plurality of smears of the sample when it is determined that the operation mode is the multi-smear mode.

In some embodiments, the sampling portion includes a sampling mechanism configured to collect the sample in a container by puncturing the container. The sampling portion may include a sampling needle.

In some embodiments, the sampling mechanism includes a drive component and a sampling component, and the drive component is configured to control the sampling component to move and collect the sample by puncturing the container according to a drive signal.

In some embodiments, the sampling portion is connected to the sample loading mechanism through a pipeline. The sampling component collects the sample by puncturing a container body of the container, and transfers the sample to the sample loading mechanism through the pipeline. The sample loading mechanism may include a blood dripping needle.

In some embodiments, the sampling portion and the sample loading mechanism are the same mechanism (such as a blood dripping needle), and the sampling portion or the sample loading mechanism collects the sample through an opening of the container, and loads the sample onto the blank slide.

Optionally, the control portion may be configured to determine the operation mode of the smear preparation portion from the single-smear mode and the multi-smear mode according to the smearing instruction, or the control portion may be configured to determine the operation mode of the smear preparation portion from the single-smear mode and the multi-smear mode according to the analysis result of the sample.

Optionally, the control portion is further configured to control the sample loading mechanism to load the sample onto the blank slide moved to the operating position by the slide loading mechanism, to prepare a single smear of the sample, when it is determined that the operation mode is the single-smear mode.

The control portion is further configured to control the sample loading mechanism to continuously load the sample onto at least two blank slides moved to the operating position by the slide loading mechanism, to prepare a plurality of smears of the sample, when it is determined that the operation mode is the multi-smear mode.

In an embodiment, when it is determined that the operation mode is the multi-smear mode, the slide loading mechanism is configured to move the first blank slide to the operating position. The control portion is configured to control the sample loading mechanism to load the sample onto the first blank slide to obtain a first slide loaded with sample, and to perform subsequent steps such as smearing on the first slide to obtain the first smear and push it out. The slide loading mechanism is configured to move the second blank slide to the operating position to prepare the second smear, and so on until the preparation of a plurality of smears is completed.

Optionally, the control portion is further configured to receive a smearing instruction, and determine the operation mode of the smear preparation portion according to the received smearing instruction.

In some embodiments, the operator may directly determine the smearing instruction according to the sample analysis result obtained by testing the sample by any blood analysis apparatus, and input the smearing instruction to the control portion of the smear preparation apparatus. The control portion switches the operation mode of the smear preparation portion to the operation mode instructed by the smearing instruction according to the smearing instruction.

In some embodiments, the control portion may receive a smearing instruction sent by the control apparatus 140 in the sample analysis system 100 according to the embodiments of the present invention, and switch the operation mode of the smear preparation portion to the operation mode indicated by the smearing instruction.

Optionally, the control apparatus 140 in the sample analysis system 100 according to the embodiments of the present invention may include the control portion of the smear preparation apparatus.

Optionally, the control portion is further configured to receive the sample analysis result of the sample, and determine the operation mode of the smear preparation portion according to the sample analysis result.

In some embodiments, the control portion acquires the sample analysis result from the blood analysis apparatus 110 in the sample analysis system 100, determines the operation mode of the smear preparation portion according to the sample analysis result, and switches the operation mode of the smear preparation portion to the operation mode determined by the control portion.

Optionally, the control portion is further configured to: in response to the sample analysis result indicating that the number of predetermined cells in the sample is lower than a threshold, determine that the smear preparation portion operates in the multi-smear mode. Furthermore, the predetermined cells are white blood cells.

Optionally, the control portion is further configured to determine that the smear preparation portion operates in the multi-smear mode when the sample analysis result indicates that the number of white blood cells in the sample is less than a first threshold, and control the sample loading mechanism of the smear preparation portion to continuously load the sample onto a first number of blank slides.

Optionally, the control portion is further configured to: determine that the smear preparation portion operates in the multi-smear mode when the sample analysis result indicates that the number of white blood cells in the sample is greater than or equal to the first threshold and less than a second threshold; and control the sample loading mechanism of the smear preparation portion to continuously load the sample onto a second number of blank slides, the first number being greater than the second number.

Specifically, the control portion receives the sample analysis result, and compares the number of white blood cells (WBCs) in the sample analysis result with the first threshold and the second threshold. If the number of WBCs is less than the first threshold, the control portion determines to prepare the first number of smears. If the number of WBCs is greater than or equal to the first threshold and less than the second threshold, the control portion determines to prepare a second number of smears. Then, the control portion switches the operation mode of the smear preparation portion to the multi-smear mode. When the first or second number of smears are prepared, the slide loading mechanism moves the first blank slide to the operating position, the sample loading mechanism loads the sample onto the first blank slide, and after smearing, drying and other steps, the first smear is obtained and pushed out. The slide loading mechanism moves the second blank slide to the operating position, and continues the preparation of the second smear, and so on, until the preparation of the first number or the second number of smears is completed.

Optionally, the control portion is further configured to: determine that the smear preparation portion operates in the multi-smear mode when the sample analysis result indicates that an infection abnormality occurs in the sample. The infection abnormality of the sample is a Plasmodium infection.

Optionally, when the control portion determines that the operation mode is the multi-smear mode, the smear preparation portion prepares at least two smears of the sample under different preparation conditions.

Optionally, the different preparation conditions include different smearing thicknesses, and the control portion controls the smear preparation portion to prepare at least two smears with different thicknesses.

Optionally, the control portion is further configured to perform at least one of the following operations.

The control portion is configured to control the sample loading mechanism to load different blood dripping volumes onto at least two blank slides moved to the operating position by the slide loading mechanism.

The control portion is configured to control the smearing mechanism to smear the samples on the at least two blank slides with different smearing angles and/or different smearing speeds respectively, to prepare at least two smears of the samples with different thicknesses.

The more the blood dripping volume, the larger the smearing angle, and the larger the smearing speed, the thicker the film on the prepared smear; the less the blood dripping volume, the smaller the smearing angle, and the smaller the smearing speed, the thinner the film on the prepared smear.

Optionally, the smear preparation portion further includes a staining mechanism. The control portion is further configured to: control the staining mechanism to stain the plurality of smears under different staining conditions when it is determined that the operation mode is the multi-smear mode. Furthermore, the different staining conditions include different pre-staining treatment conditions. The pre-staining treatment condition includes a methanol fixation treatment or a distilled water hemolysis treatment with a staining mechanism.

In some embodiments, the control portion may further be configured to control the smear preparation portion to prepare a first smear of the sample under a first staining condition and to prepare a second smear of the sample under a second staining condition, when the sample analysis result indicates that Plasmodium infection occurs in the sample, a sample smearing thickness of the first smear being smaller than the sample smearing thickness of the second smear. The first staining condition includes a methanol fixation treatment on the first smear, and the second staining condition includes a distilled water hemolysis treatment on the second smear.

Specifically, the control portion determines a presence of Plasmodium infection in the sample according to the sample analysis result, determines that at least one thin smear and at least one thick smear need to be prepared for the sample, and switches the operation mode of the smear preparation portion to the multi-smear mode. When the smear preparation portion prepares a thin smear, the slide loading mechanism moves a blank slide to the operating position, the sample loading mechanism loads a first blood dripping volume of the sample onto the blank slide, and the smearing mechanism smears the sample on the slide at a first smearing angle and first smearing speed. Then, the sample on the slide is fixed with methanol before staining to maintain the morphology of the red blood cells in the sample on the slide. The morphology of Plasmodium in the red blood cell remains intact, which facilitates determination of the species, morphology and life stage of Plasmodium. Then, after the fixation treatment before staining, the sample on the slide is dried, and then stained with Giemsa stain or Wright stain to obtain the thin smear. When the smear preparation portion prepares a thick smear, the slide loading mechanism moves a blank slide to the operating position, the sample loading mechanism loads a second blood dripping volume of the sample onto the blank slide, and the second blood dripping volume is larger than the first blood dripping volume. The smearing mechanism smears the sample on the slide with a second smearing angle and a second smearing speed, the second smearing angle is greater than the first smearing angle, and the second smearing speed is faster than the first smearing speed. Then, distilled water is used to perform hemolysis treatment on the sample on the slide before staining to lyse the red blood cells, which facilitates counting the number of Plasmodium. Then, after the distilled water hemolysis treatment before staining, the sample on the slide is dried, and stained with Giemsa stain or Wright stain to obtain the thick smear.

In some embodiments, the sample may be in the form of a thin film in a thin smear and in the form of a disk in a thick smear. The cells on the thin smear are laid flat on the slide, which is convenient for observing the species, morphology and life stage of Plasmodium, and the cells on the thick smear are stacked on the slide, which is convenient for comprehensive counting of Plasmodium.

Optionally, the different staining conditions include different staining parameters. Furthermore, the staining parameter may include: a staining time, a staining temperature, types of reagents in the stain and their proportions. The type of reagent may include a staining reagent or a buffering reagent. For example, the i-th staining parameter may include the i-th staining time, the i-th staining temperature, and a ratio of the reagent Sᵢ₁ to the reagent Sᵢ₂ in the i-th stain as Pᵢ₁:Pᵢ₂, i being a positive integer. The longer the staining time, the more the white blood cells bind to the stain, and the darker the white blood cells are. In the stain containing the staining reagent and the buffering reagent, the ratio of the staining reagent to the buffering reagent can be adjusted. The higher the ratio of the staining reagent to the buffering reagent, the darker the white blood cells stained, and vice versa. The higher the staining temperature, the darker the cells stained.

In some embodiments, the control portion may preset a plurality of different staining parameters, and the control portion may automatically select the required staining parameters for staining the sample according to the sample analysis result. The staining parameters may be set or updated as needed (such as increase, decrease, change, etc.).

In some embodiments, the staining parameter may further include a staining mode. The staining mode includes drip staining, dip staining of a single smear, and jar staining of multiple smears. When the smear preparation portion is in the drip staining mode, the stain can be dripped onto a single smear for staining. When a plurality of smears are prepared, the staining time and stains required for respective smears are different. Therefore, when the plurality of smears are stained with the drip staining mode, one or more of the stain, staining time, and staining temperature may be changed at any time according to the needs of each smear. When the smear preparation portion is in the mode for dip staining of a single smear, the smear may be put into a staining box, and the corresponding stain is added to the staining box for staining. Similarly, when the plurality of smears are stained with the mode for dip staining of a single smear, one or more of the stain, staining time and staining temperature in the staining box may be changed at any time according to the needs of each smear on the staining time and the stain. When the smear preparation portion is in the mode for jar staining of multiple smears, a plurality of smears are put in one jar for staining. At this time, only the staining time can be adjusted until the staining of the plurality of smears is completed. After that, the stain and staining time in the jar can be changed to stain a plurality of smears in the next staining process. Furthermore, the smear preparation portion can be switched between different staining modes.

In an embodiment, different staining parameters (the number of the staining parameters is a) are preset in the control portion, where b0 is a normal staining parameter, and b0 may include a first staining time, a first staining temperature, a first stain and a drip staining mode. The control portion determines that the number of white blood cells in the sample analysis result is low, the sample belongs to a low-value white blood cell sample, and m smears need to be prepared. One smear is stained normally, that is, stained by using the normal staining parameter b0, and the other smears are stained with the staining parameter b1, which may include a second staining time, a second staining temperature, a second stain and a drip staining mode. The control portion sends the smearing instruction to the smear preparation portion and the smearing instruction includes: preparing m smears of the sample, the staining parameter for one smear is b0, and the staining parameter for the other m-1 smears is b1. The smear preparation portion receives the smearing instruction, loads m slides and adds the sample to the m slides, performs staining according to the staining parameters in the smearing instruction, and then washes and air-dries the stained smears to obtain m smears of the sample.

In some embodiments, the control portion may further determine the number of smears to be prepared and the staining parameter for the prepared smear, including the staining parameter and pre-staining treatment condition, according to the sample analysis result.

In an embodiment, c different staining parameters are preset in the control portion, where the first staining parameter d0 may include the treatment of maintaining the morphology of red blood cells, a third staining time, a third staining temperature, a third stain and a drip staining mode, and the second staining parameter d1 may include the treatment of lysing red blood cells, a fourth staining time, a fourth staining temperature, a fourth stain and a drip staining mode. The control portion determines that the sample is infected with Plasmodium according to the sample analysis result, m1 thick smears and m2 thin smears are required to be prepared, m1 thick smears are stained with the second staining parameter d1, and m2 thin smears are stained with the first staining parameter d0. Then the control portion sends a smearing instruction to the smear preparation portion, and the smearing instruction includes: preparing m1 thick smears and m2 thin smears of the sample, the preparation conditions of m1 thick smears including the first smearing thickness and the second staining parameter d1, and the preparation conditions of m2 thin smears including the second smear thickness and the first staining parameter d0. The smear preparation portion receives the smearing instruction, loads m1 slides and adds the sample on the m1 slides. After that, smearing is performed based on the first smearing thickness, then the smears are stained with the second staining parameter d1, and then the stained smears are washed and air-dried to obtain m1 thick smears of the sample. The smear preparation portion further loads m2 slides and adds the sample on the m2 slides. Then, smearing is performed based on the second smearing thickness, the smears are stained with the first staining parameter d0, and then the stained smears are washed and air-dried to obtain m2 thin smears of the sample.

Optionally, the apparatus further includes a cell image analysis portion.

The control portion is configured to control the cell image analysis portion to photograph the plurality of smears under different photographing conditions when it is determined that the operation mode is the multi-smear mode, and the different photographing conditions include at least one of different photographing positions, different photographing areas, different photographing numbers, or different photographing lenses.

In some embodiments, the photographing position includes at least one of: a tail, a head, a body, a head-body junction, a body-tail junction, an edge, or a designated part of the sample on the smear.

In some embodiments, the photographing area includes an area of the sample on the smear photographed each time. In an embodiment, when photographing is performed with an oil immersion lens, the area of the sample seen through the oil immersion lens may be referred to as a field of view of the oil immersion lens. When the oil immersion lenses with different magnifications are used to observe the sample, the fields of view of the oil immersion lenses will be different. The larger the magnification of the oil immersion lens, the smaller the field of view of the oil immersion lens.

In some embodiments, the different photographing lenses include different optical parameters of the photographing lenses. Furthermore, the optical parameter includes magnification. As illustrated in FIG. 5, the different photographing lenses may include a first objective lens, a second objective lens and an eyepiece lens. The first objective lens may be, for example, a 10× objective lens, and the second objective lens may be, for example, a 100x objective lens. The photographing lenses may further include a third objective lens, and the third objective lens may be, for example, a 40x objective lens. In an embodiment, images of at least 200 fields of view of the oil immersion lens are required to be photographed for the thick smear.

In some embodiments, the photographed number includes a number of cells photographed in the smear.

In some embodiments, the cell image analysis portion may have a default photographing condition. The control portion may control the cell image analysis portion to photograph and analyze the smear output by the smear preparation portion based on the default photographing condition.

In an embodiment, when the preset condition includes that the number of white blood cells is lower than a threshold, the control portion determines the number of smears to be prepared according to the sample analysis result, such as 3, and controls the smear preparation portion to switch to the multi-smear mode to prepare 3 smears of the sample. Then the three smears are sent to the cell image analysis portion for photographing and analysis. The cell image analysis portion can photograph the smears of the sample based on the default photographing condition to obtain cell images, and identify and classify the cell images to obtain the corresponding identification and classification result as the cell analysis result. The cell image analysis portion saves the cell analysis result locally.

It should be understood that the default photographing condition may be set or changed by the operator as required, which is not limited herein.

In some embodiments, when the sample result indicates that Plasmodium infection occurs in the sample, the control portion controls the smear preparation portion to prepare a first smear and a second smear of the sample, and the sample smearing thickness of the first smear is smaller than the sample smearing thickness of the second smear. The control portion controls the cell image analysis portion to photograph a cell image of a first area of the first smear and a cell image of a second area of the second smear, and the first area is smaller than the second area.

In an embodiment, when the preset condition includes Plasmodium infection, the control portion determines that Plasmodium infection occurs in the sample according to the sample analysis result, controls the smear preparation portion to prepare a thick smear and a thin smear, and sends the prepared thick smear and thin smear to the cell image analysis portion for photographing and analysis, which is specifically as follows. When the control portion controls the cell image analysis portion to photograph the image of the thick smear, the photographing position includes at least the edge of the sample on the smear, an objective lens with a lower magnification, such as the first objective lens with a magnification of 10 times, is adopted, and images of at least m fields of view of the objective lens are photographed to cover the entire sample region on the thick smear, m being a positive integer. When the control portion controls the cell image analysis portion to photograph the image of the thin smear, the photographing position of the thin smear includes at least the tail, head, body, head-body junction, body-tail junction, edge of the sample on the smear, an objective lens with a higher magnification, such as the second objective lens with a magnification of 100 times, is adopted, and images of at least n fields of view of the objective lens are photographed to cover the entire sample region on the thin smear, n being a positive integer. The cell image analysis portion photographs the images of at least m fields of view of the objective lens on the thick smear and the images of at least n fields of view of the objective lens on the thin smear. Next, the user can determine the species and stage of Plasmodium and estimate a concentration of Plasmodium in the sample by observing and analyzing the thin smear images. The user counts the number of Plasmodium when a certain number of white blood cells are observed based on the thin smear. The cell image analysis portion can calculate the number of Plasmodium per cubic millimeter of the sample based on the total number of white blood cells and the counted number of Plasmodium or the estimated concentration of Plasmodium in the sample analysis result. The cell image analysis portion may output a cell analysis result, including the number of Plasmodium per cubic millimeter of the sample, the concentration of Plasmodium in the sample, and the like. The cell image analysis portion may store the cell analysis result locally.

An embodiment of the present invention further provides a computer-readable storage medium having stored therein a plurality of program instructions that, when being called and executed by a processor or a processing component, cause the processor or the processing component to execute some or all of the steps or any combination of the steps in the sample analysis method in the various embodiments of the present invention.

In the smear preparation apparatus, sample analysis system and method according to the embodiments of the present invention, smears are prepared in different modes for cell analysis according to the abnormal condition in the sample analysis result, a richer database is provided for the data source of cell analysis, thereby improving the accuracy and efficiency of cell analysis, and saving labor costs and resources greatly.

It will be understood by those skilled in the art that all features disclosed in this specification (including accompanying claims, abstract and drawings) and all processes or elements of any method or apparatus so disclosed may be combined in any manner, except that the features are mutually exclusive. Each feature disclosed in this specification (including the accompanying claims, abstract and drawings) may be replaced by an alternative feature serving the same, equivalent or similar purpose, unless otherwise expressly stated.

Furthermore, it will be understood by those skilled in the art that although some of the embodiments described herein include some features included in other embodiments but not others, combinations of features of different embodiments are intended to be within the scope of the invention and form different embodiments. For example, in the claims, any of the claimed embodiments may be used in any combination.

The technical terms used in the embodiments of the present invention are only used to describe specific embodiments and are not intended to limit the present invention. As used herein, the singular forms "a" "said" and "the" will cover the expression in the plural forms as well, unless the context clearly dictates otherwise. Furthermore, the use of "including" and/or "comprising" in this specification refers to the presence of the stated features, integers, steps, operations, elements and/or components, but does not preclude the presence or addition of one or more other features, integers, steps, operations, elements and/or components.

The corresponding structures, materials, actions, and equivalents (if any) of all apparatuses, steps and function elements in the appended claims are intended to include any structure, material, or action for performing the function in combination with other explicitly claimed elements. The description of the present invention has been presented for purposes of illustration and description, but is not intended to be exhaustive or to limit the present invention to the form disclosed. Various modifications and variations will be apparent to those skilled in the art without departing from the scope and spirit of the present invention. The embodiments described in the present invention can better disclose the principles and practical applications of the present invention, and enable those skilled in the art to understand the present disclosure.

The flowchart described in the present invention is only an embodiment, and various modifications and changes may be made to this illustration or the steps in the present invention without departing from the spirit of the present invention. For example, these steps may be performed in a different sequence, or certain steps may be added, deleted, or modified. Those skilled in the art would understand that all or part of the flow of the above embodiments, and the equivalent changes made according to the claims of the present invention shall fall within the scope of the present invention.

## Claims

1. A sample analysis system, **characterized in that** the sample analysis system comprises:
a blood analysis apparatus, configured to test a sample and obtain a sample analysis result;
a smear preparation apparatus, configured to prepare a smear for the sample;
a cell image analysis apparatus, configured to photograph and analyze the smear;
a transfer apparatus, comprising a first transfer track configured to transfer the sample from the blood analysis apparatus to the smear preparation apparatus, and a second transfer track configured to transfer the smear from the smear preparation apparatus to the cell image analysis apparatus; and
a control apparatus communicatively connected with the blood analysis apparatus, the smear preparation apparatus, the cell image analysis apparatus, and the transfer apparatus, and configured to:
acquire the sample analysis result of the sample from the blood analysis apparatus; and
control the first transfer track to transfer the sample to the smear preparation apparatus, control the smear preparation apparatus to prepare at least two smears of the sample, and control the second transfer track to transfer the at least two smears to the cell image analysis apparatus for photographing and analysis, when the sample analysis result satisfies a preset condition.

2. The system of claim 1, wherein the preset condition comprises an abnormal number of predetermined cells in the sample.

3. The system of claim 2, wherein the abnormal number of predetermined cells is a number of predetermined cells that is below a threshold.

4. The system of claim 2 or 3, wherein the predetermined cells are white blood cells.

5. The system of any one of claims 1 to 4, wherein the control apparatus is further configured to:
control the smear preparation apparatus to prepare a first number of smears of the sample when the sample analysis result indicates that a number of white blood cells in the sample is less than a first threshold;
control the smear preparation apparatus to prepare a second number of smears of the sample when the sample analysis result indicates that a number of white blood cells in the sample is greater than or equal to the first threshold and less than a second threshold, the first number being greater than the second number.

6. The system of claim 5, wherein the control apparatus is further configured to:
control the smear preparation apparatus to prepare a third number of smears of the sample when the sample analysis result indicates that a number of white blood cells in the sample is greater than or equal to the second threshold and less than a third threshold, the second number being greater than the third number.

7. The system of any one of claims 1 to 6, wherein the preset condition comprises abnormal infection of the sample.

8. The system of claim 7, wherein the abnormal infection of the sample is Plasmodium infection.

9. The system of any one of claims 1 to 8, wherein the control apparatus is further configured to: control the smear preparation apparatus to prepare at least two smears of the sample under different preparation conditions, when the sample analysis result satisfies the preset condition.

10. The system of claim 9, wherein the different preparation conditions comprise different smearing thicknesses, and the control apparatus is further configured to:
control the smear preparation apparatus to prepare the at least two smears of the sample with different smearing thicknesses, when the sample analysis result satisfies the preset condition.

11. The system of claim 9, wherein the control apparatus is further configured to:
control the smear preparation apparatus to prepare the at least two smears of the sample with different smearing thicknesses under at least one of different blood dripping volumes, different smearing angles or different smearing speeds.

12. The system of any one of claims 9 to 11, wherein the different preparation conditions comprise different staining conditions.

13. The system of claim 12, wherein the different staining conditions comprise different pre-staining treatment conditions.

14. The system of claim 13, wherein the pre-staining treatment condition comprises a treatment for maintaining morphology of red blood cells or a treatment for lysing red blood cells.

15. The system of any one of claims 12 to 14, wherein the control apparatus is further configured to:
control the smear preparation apparatus to prepare a first smear of the sample under a first staining condition and to prepare a second smear of the sample under a second staining condition when the sample analysis result indicates that Plasmodium infection occurs in the sample, wherein a sample smearing thickness of the first smear is smaller than that of the second smear, the first staining condition comprises a methanol fixation treatment on the first smear, and the second staining condition comprises a distilled water hemolysis treatment on the second smear.

16. The system of any one of claims 1 to 15, wherein the control apparatus is further configured to:
control the cell image analysis apparatus to photograph the at least two smears under different photographing conditions, when the sample analysis result satisfies the preset condition.

17. The system of claim 16, wherein the different photographing conditions comprise at least one of different photographing positions, different photographing areas, different photographing numbers, or different photographing lenses.

18. The system of claim 16 or 17, wherein the control apparatus is further configured to:
control the smear preparation apparatus to prepare a first smear and a second smear of the sample when the sample analysis result indicates that Plasmodium infection occurs in the sample, wherein a sample smearing thickness of the first smear is smaller than that of the second smear; and
control the cell image analysis apparatus to photograph a cell image of a first area of the first smear and a cell image of a second area of the second smear, wherein the first area is smaller than the second area.

19. The system of any one of claims 1 to 18, wherein the control apparatus is provided independently of the blood analysis apparatus, the smear preparation apparatus and the cell image analysis apparatus.

20. The system of any one of claims 1 to 19, wherein the sample is a blood sample or a body fluid sample.

21. The system of any one of claims 1 to 20, wherein the control apparatus is further configured to:
control the smear preparation apparatus to prepare a smear of the sample, and control the second transfer track to transfer the smear to the cell image analysis apparatus for photographing and analysis, when the sample analysis result does not satisfy the preset condition.

22. A smear preparation apparatus, **characterized in that** the smear preparation apparatus comprises:
a sampling portion, configured to collect a sample;
a smear preparation portion, configured to prepare the sample collected by the sampling portion into a smear, wherein the smear preparation portion comprises a slide loading mechanism configured to move a blank slide to an operating position, a sample loading mechanism configured to load the sample onto the blank slide and a smearing mechanism configured to smear the sample on the blank slide, and the smear preparation portion is configured with a single-smear mode and a multi-smear mode; and
a control portion, electrically connected to the sampling portion and the smear preparation portion, and configured to:
determine an operation mode of the smear preparation portion from the single-smear mode and the multi-smear mode; and
control the smear preparation portion to prepare a single smear of the sample when it is determined that the operation mode is the single-smear mode; and control the smear preparation portion to prepare a plurality of smears of the sample when it is determined that the operation mode is the multi-smear mode.

23. The apparatus of claim 22, wherein the control portion is further configured to:
control the sample loading mechanism to load the sample onto a blank slide moved to the operating position by the slide loading mechanism to prepare the single smear of the sample, when it is determined that the operation mode is the single-slide mode;
control the sample loading mechanism to continuously load the sample onto at least two blank slides moved to the operating position by the slide loading mechanism to prepare the plurality of smears of the sample, when it is determined that the operation mode is the multi-smear mode.

24. The apparatus of claim 22 or 23, wherein the control portion is further configured to receive a smearing instruction, and determine the operation mode of the smear preparation portion according to the received smearing instruction.

25. The apparatus of claim 22 or 23, wherein the control portion is further configured to receive a sample analysis result of the sample, and determine the operation mode of the smear preparation portion according to the sample analysis result.

26. The apparatus of claim 25, wherein the control portion is further configured to:
determine that the smear preparation portion operates in the multi-smear mode, when the sample analysis result indicates that a number of predetermined cells in the sample is lower than a threshold.

27. The apparatus of claim 26, wherein the predetermined cells are white blood cells, and the control portion is further configured to:
determine that the smear preparation portion operates in the multi-smear mode, and control the sample loading mechanism of the smear preparation portion to continuously load the sample onto a first number of blank slides, when the sample analysis result indicates that a number of white blood cells in the sample is less than a first threshold;
determine that the smear preparation portion operates in the multi-smear mode, and control the sample loading mechanism of the smear preparation portion to continuously load the sample onto a second number of blank slides, when the sample analysis result indicates that a number of white blood cells in the sample is greater than or equal to the first threshold and less than a second threshold, wherein the first number is greater than the second number.

28. The apparatus of any one of claims 25 to 27, wherein the control portion is further configured to:
determine that the smear preparation portion operates in the multi-smear mode, when the sample analysis result indicates that an infection abnormality occurs in the sample.

29. The apparatus of claim 28, wherein the infection abnormality of the sample is Plasmodium infection.

30. The apparatus of any one of claims 22 to 29, wherein the smear preparation portion is configured to prepare at least two smears of the sample under different preparation conditions, when the control portion determines that the operation mode is the multi-smear mode.

31. The apparatus of claim 30, wherein the different preparation conditions comprise different smearing thicknesses, and the control portion is further configured to control the smear preparation portion to prepare the at least two smears with different thicknesses.

32. The apparatus of claim 31, wherein the control portion is further configured to:
control the sample loading mechanism to load different blood dripping volumes onto at least two blank slides moved to the operating position by the slide loading mechanism; and/or
control the smearing mechanism to smear the sample onto at least two blank slides with different smearing angles and/or different smearing speeds respectively, to prepare at least two smears of the sample with different thicknesses.

33. The apparatus of any one of claims 22 to 32, wherein the smear preparation portion further comprises a staining mechanism, and the control portion is further configured to:
control the staining mechanism to stain the plurality of smears under different staining conditions when it is determined that the operation mode is the multi-smear mode.

34. The apparatus of claim 33, wherein the different staining conditions comprise different pre-staining treatment conditions.

35. The apparatus of claim 34, wherein the pre-staining treatment condition comprises a methanol fixation treatment or distilled water hemolysis treatment with the staining mechanism.

36. The apparatus of any one of claims 22 to 35, further comprising a cell image analysis portion, and the control portion is further configured to:
control the cell image analysis portion to photograph the plurality of smears under different photographing conditions when it is determined that the operation mode is the multi-smear mode, wherein the different photographing conditions comprise at least one of different photographing positions, different photographing areas, different photographing numbers, or different photographing lenses.

37. A sample analysis method, **characterized in that** the method comprises:
acquiring a sample analysis result of a sample from a blood analysis apparatus; and
when the sample analysis result satisfies a preset condition, transferring, by a first transfer track, the sample to a smear preparation apparatus; preparing, by the smear preparation apparatus, at least two smears of the sample; transferring, by a second transfer track, the at least two smears to a cell image analysis apparatus; and photographing and analyzing, by the cell image analysis apparatus, cell images of the at least two smears.

38. The method of claim 37, wherein the preset condition comprises an abnormal number of predetermined cells in the sample and/or abnormal infection of the sample.

39. The method of claim 38, further comprising:
preparing, by the smear preparation apparatus, a first number of smears of the sample, when the sample analysis result indicates that a number of white blood cells in the sample is less than a first threshold; and
preparing, by the smear preparation apparatus, a second number of smears of the sample, when the sample analysis result indicates that a number of white blood cells in the sample is greater than or equal to the first threshold and less than a second threshold, wherein the first number is greater than the second number.

40. The method of claim 37 or 38, further comprising:
preparing, by the smear preparation apparatus, the at least two smears of the sample under different preparation conditions when the sample analysis result satisfies the preset condition.

41. The method of claim 40, further comprising:
photographing, by the cell image analysis apparatus, the at least two smears under different photographing conditions when the sample analysis result satisfies the preset condition.

42. A computer-readable storage medium having stored therein a program that, when executed by a processor, causes the processor to implement the method of any one of claims 37 to 41.
